Europäisches Patentamt

European Patent Office

Office européen des brevets

(19)

(11) Numéro de publication: **0 102 887**

**B1**

(12) **FASCICULE DE BREVET EUROPEEN**

(45) Date de publication du fascicule du brevet: 02.09.87

(51) Int. Cl.⁴: **A 61 B 3/02**

(21) Numéro de dépôt: **83401616.4**

(22) Date de dépôt: **05.08.83**

(54) **Appareil de dépistage des défauts de la vue et cassette pour le service d'un tel appareil.**

(30) Priorité: **27.08.82 FR 8214745**

(43) Date de publication de la demande:
**14.03.84 Bulletin 84/11**

(45) Mention de la délivrance du brevet:
**02.09.87 Bulletin 87/36**

(84) Etats contractants désignés:
**DE FR GB IT NL SE**

(56) Documents cités:
**FR-A-2 301 215**
**FR-A-2 437 818**
**GB-A-1 385 675**
**US-A-2 798 408**
**US-A-3 684 355**
**US-A-3 861 790**
**US-A-3 891 311**

(73) Titulaire: **ESSILOR INTERNATIONAL Cie
Générale d'Optique
1 Rue Thomas Edison Echat 902
F-94028 Creteil Cedex (FR)**

(72) Inventeur: **Capo-Gual, Christine
4 Avenue Parmentier
F-93270 Sevran (FR)**
Inventeur: **Delattre, Luc
724 Quai de la Libération
F-60700 Pont Saint Maxence (FR)**
Inventeur: **Hennequin, Jean-Claude
2 rue du Stade Congis Sur Therouanne
F-77440 Lizy Sur Ourcq (FR)**

(74) Mandataire: **Hirsch, Marc-Roger
Cabinet Hirsch 34 rue de Bassano
F-75008 Paris (FR)**

Courier Press, Leamington Spa, England.

# Description

La présente invention s'applique à un appareil d'autotest pour l'examen de la vue d'un patient afin de dépister les défaillances visuelles et les défauts de vision de chacun des yeux de ce patient par la vision d'images-tests successives présentées pour la vision de près, la vision de loin et la vision intermédiaire à l'aide d'un système de miroirs et/ou de lentilles. Elle a plus particulièrement pour objet un appareil d'autotest pour l'examen de la vue des deux yeux d'un patient et l'auto-dépistage de ses défaillances visuelles sans intervention d'un opérateur.

Il est connu d'utiliser des appareils de dépistage des défauts de la vue en vision de loin et en vision de près consistant à examiner des images-tests logées à l'intérieur du boîtier de l'appareil, via un circuit optique comportant des lentilles ou, de préférence, un ou plusieurs miroirs et lames semi-réfléchissantes inclinés ou des prismes. Les images-tests utilisées peuvent être portées par des tambours ou des plateaux rotatifs et un système de caches ou de volets peut venir démasquer le circuit optique ou les images que l'on désire utiliser pour l'examen de la vue du patient.

Ces appareils de dépistage qui permettent un bon examen de la vue, même en vision stéréoscopique, présentent néanmoins diverses insuffisances par rapport aux conditions modernes d'examen de la vue. En effet, selon l'âge des patients et les types de défauts de vision à détecter, il est nécessaire de disposer parfois d'un grand nombre d'images-tests qui ne peuvent pas toutes être logées sur les tambours ou disques rotatifs. Pour l'examen de la vue d'un patient, il est par ailleurs indispensable avec les appareils connus, de faire suivre ce patient par un opérateur au cours du test, ce qui augmente beaucoup le coût d'un test approfondi de décourage de nombreux patients de passer ou de renouveler un test de vision à cause des contraintes financières et physiques de cet examen. Il en est ainsi dans le cas des appareils selon les brevets US—A—2 798 408 et FR—A—2 437 818.

Il faut remarquer selon ce US—A—2 798 408 (col. 7, 1. 45 et suivantes) que, pour une distance axiale déterminée entre yeux du patient et le test présenté, il n'existe que deux positions axiales d'un système optique de lentilles permettant l'observation par le patient suivant le même angle (α) en vision de loin et en vision de près. En fait, les lentilles doivent être déplacées par intervention manuelle pour obtenir les images respectives sous le même angle de vision. C'est par basculement de l'appareil et modification axiale des systèmes optiques que l'on passe dans l'appareil de ce USP de la présentation des tests de vision de loin aux tests de vision de près. Cette intervention, manuelle, n'est pas nécessaire dans l'appareil selon l'invention.

L'un des buts de la présente invention est précisément de permettre de réduire le coût d'un dépistage approfondi de la vue et de permettre de le réaliser dans de meilleures conditions de confort pour le patient, la présentation des tests étant programmée à l'avance en fonction du patient dont on veut contrôler la vue.

La présente invention a pour objet un tel appareil d'autotest caractérisé en en ce qu'il comporte des miroirs et/ou lentilles interposés sur au moins trois voies de vision distinctes, chaque voie étant définie par ses propres dispositifs optiques, ces voies de vision comprenant une voie pour l'examen de la vision de loin, au moins une voie pour l'examen de la vision intermédiaire et une voie pour l'examen de la vision de près, et en ce qu'il comporte une chambre de vision munie d'un appui tête réglable pour le positionnement du visage d'un patient, permettant à celui-ci par balayage vertical du regard, l'observation successive des trois images-tests, appartenant chacune à une des trois voies de vision.

Selon un mode particulier de réalisation de l'invention, à l'entrée dans la chambre de vision les voies de vision sont placées de façon telle que la voie pour l'examen de la vision de loin soit la plus haute, la voie pour l'examen de la vision de près soit la plus basse et la voie pour l'examen de la vision intermédiaire soit en position intermédiaire.

En fait, l'appareil décrit dans le US—A—2 798 408 ainsi que celui décrit dans FR—A—2 437 818 ne comportent qu'une unique voie de vision et la même voie optique est employée que ce soit en vision de près ou en vision de loin, grâce à la modification de la position axiale des éléments optiques qu'il contient.

Lorsque l'appareil comporte au moins une voie pour la vision de loin et au moins une autre voie pour la vision à distance intermédiaire, ces voies sont équipées de miroirs sphériques concaves adaptés pour permettre la vision d'une même image-test indifféremment sur chacune des deux voies et permettre ainsi de n'utiliser sur le film ou le ruban qu'une seule image-test pour la vision de loin et à distance intermédiaire.

Selon un mode particulier de réalisation de l'invention, les images-tests sont portées par au moins un ruban ou un film monté dans un bloc à deux bobines d'enroulage logé dans une cassette fixée de façon amovible sur un support du corp de l'appareil, au moins une des bobines étant susceptible d'être entraînée en rotation pour faire défiler une batterie d'images-tests sur une partie du ruban ou du film, qui est tendue entre les deux bobines et qui se place en face d'ouvertures d'amenée des images-tests sur la voie de vision ménagée sur le corps de l'appareil.

Pour chacune des voies de vision l'appareil comporte, du côté du patient, deux ouvertures, chaque ouverture de chaque voie étant susceptible d'être occultée, individuellement ou en combinaison, avec d'autres ouvertures par un moyen d'obturation tel qu'un volet mobile.

Suivant un autre mode de réalisation de l'invention l'appareil comporte un circuit de haut-parleur couplé à la rotation des bobines de la cassette pour donner des instructions et/ou poser des questions au patient pendant le défilement de la batterie d'images-tests.

Afin de permettre au patient de réaliser lui-même la batterie de tests de vision, l'appareil peut comporter un circuit d'enregistrement des réponses du patient aux questions du circuit de haut-parleur couplé et/ou synchronisé à la rotation des bobines de cassettes.

L'exploitation des réponses du patient peut s'effectuer de façon plus aisée lorsque le circuit d'enregistrement des réponses du patient aux questions du circuit de haut-parleur comporte une bande d'enregistrement qui se déroule en synchronisme avec le film ou ruban d'images-tests.

La vitesse de défilement des tests est liée directement au rythme suivi par le patient. Un test déterminé n'apparaît que lorsque le patient a répondue à la presentation du test précédent.

On peut également prévoir un retour en arrière de la bande porte tests lorsque le patient n'a pas compris la question.

Les moyens d'occulation des ouvertures de chaque voie sont directement liés au test présenté et sont donc programmés en fonction de ce test.

Suivant un autre mode de réalisation de l'appareil selon l'invention, il comporte des sources ponctuelles ou quasi-ponctuelles de lumière, telles que des diodes LED, permettant de détecter les défauts de champ visuel du patient et les défaillances ponctuelles éventuelles de sa rétine.

Selon un autre mode encore de réalisation, l'appareil comporte une chambre de vision recevant la tête du patient et équipée d'un appui réglable pour le front du patient lui permettant de maintenir, en cours d'examen, le centre de rotation de chacun de ses yeux, confondu sensiblement avec le point de convergence des voies de vision correspondant à chaque oeil.

La chambre de vision recevant la tête du patient est de préférence équipée de moyens d'éclairage et/ou de réflexion aptes à réaliser une ambiance lumineuse permettant d'adapter les yeux du patient à des conditions d'éclairement déterminées en simulant une zone d'éclairement variable autour du test. Ceci permet de recréer artificiellement les conditions d'ambiance lumineuse du poste de travail d'un individu et d'en détecter l'influence sur la vision de celui-ci.

En disposant de niveaux d'éclairement suffisants, on peut également utiliser l'ambiance à des tests de résistance à l'éblouissement.

Selon un mode de réalisation plus élaboré de l'appareil, le rouleau ou le film de la cassette est susceptible d'être entraîné en défilement à une vitesse aisément réglable afin de permettre de tester l'aptitude du patient à visionner des images défilantes.

La cassette amovible portant le ruban ou le film peut être fixée sur le corps de l'appareil dans une position faisant défiler ce ruban ou ce film dans une direction sensiblement verticale ou horizontale par rapport aux yeux du patient, ou bien est susceptible d'être fixée sur le corps de l'appareil alternativement dans ces deux positions perpendiculaires.

Selon un autre mode de réalisation encore plus élaboré, la cassette amovible portant le ruban ou le film et fixée sur le corps de l'appareil est susceptible d'être orientée aisément en une pluralité de positions dans un plan déplacement du ruban ou du film de manière à permettre de faire défiler le ruban ou le film selon une pluralité de directions correspondantes comportant les directions verticales et horizontales par rapport aux yeux du patient ainsi qu'une pluralité de directions intermédiaires et afin de permettre le défilement du ruban ou du film dans toutes les directions, la cassette porte son propre moteur d'entraînement du ruban ou du film relié à un moyen adéquat de contrôle réglable à distance tel qu'une liaison électrique souple ou glissante.

Selon divers modes de réalisation des cassettes desservant l'appareil selon l'invention de dépistage des défauts de la vue, la cassette comporte entre ses bobines au moins un rouleau tendeur appliqué sur la partie de ruban ou de film tendue entre les deux bobines, et le corps de la cassette présente, pour chaque image-test, une ouverture de dimensions appropriées.

L'appareil peut comporter une cellule de détection de la position du ruban.

Suivant un autre mode de réalisation particulièrement pratique des cassettes pour l'appareil selon l'invention, une des bobines de la cassette est constamment rappelée en position bobinée par un ressort tel qu'un ressort spiral de torsion, tandis que l'autre bobine est reliée, au moins en position de service sur l'appareil, à un moteur de déroulement tel qu'un moteur électrique, via une transmission irréversible, de telle manière qu'en position de servivce sur l'appareil, la bande ou le film de la cassette soit constamment tendu(e) et puisse néanmoins être déplacé(e) et arrêté(e) en un point quelconque de la batterie d'images-tests.

D'autres buts, avantages et caractéristiques d'un mode de réalisation de l'appareil de détection des défauts de la vue selon l'invention et de sa cassette-type portant une batterie de tests apparaitront à la lecture de la description, faite à titre non limitatif et en regard du dessin annexé où:

la figure 1 représente une vue en coupe schématique de l'appareil selon l'invention équipé de sa cassette d'utilisation;

la figure 2 est une vue selon la flèche B de la figure 1, de la chambre de vision de l'appareil de la figure 1;

la figure 3 est une vue de dessus, en coupe partielle, selon la flèche A de la figure 1, de la chambre de vision de l'appareil;

la figure 4 est une vue de dessus, à plus grande échelle, de la cassette équipant l'appareil selon l'invention, face frontale enlevée;

la figure 5 est une vue en coupe partielle de l'une des bobines de la cassette selon la figure 4; et

la figure 6 est une vue en coupe de l'autre bobine de la cassette.

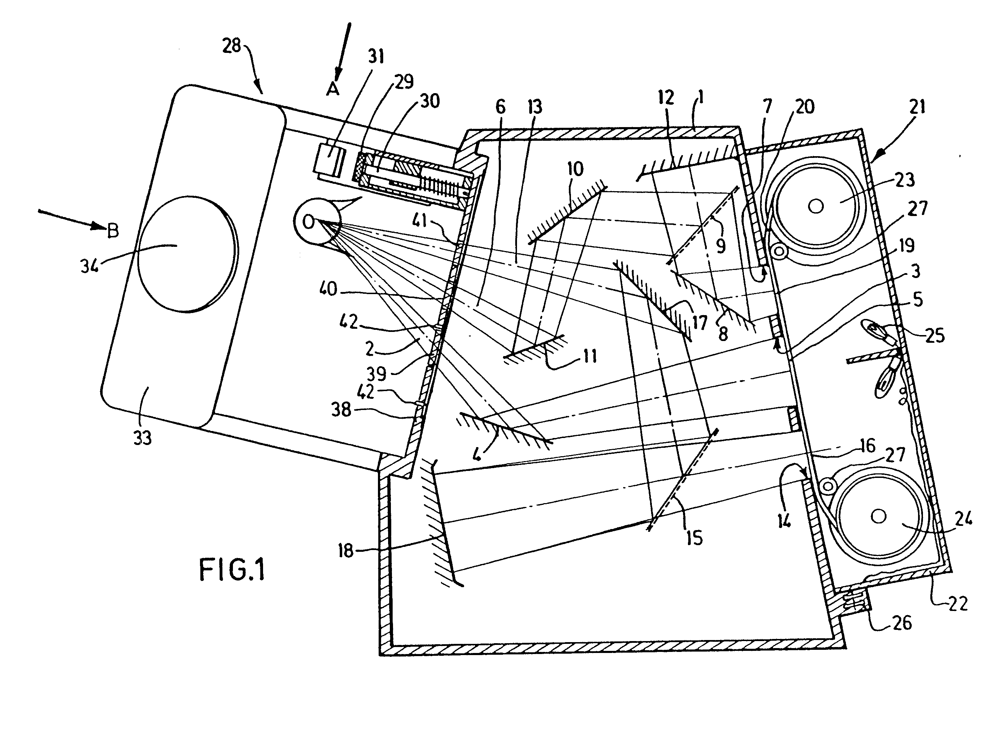

Si l'on se reporte à la figure 1, on voit que l'appareil selon l'invention est monté dans un corps 1 en un matériau adéquat tel que de la matière plastique et qui comporte sur des supports internes plusieurs circuits ou voies de vision différents qui aboutissent tous aux centres 0 de chacun des yeux du patient à examiner. La voie de vision de près 2 part d'une ouverture 3 ménagée dans le corps de l'appareil et est renvoyée sur un miroir plan 4 pour donner un parcours d'environ 30 à 40 centimètres pour les rayons lumineux issus d'une image 5 en vision de près. La voie 6 de vision à distance intermédiaire comporte, à partir d'un ouverture 7 dans le corps 1, une série de miroirs plans 8, 10, 11, sur le parcours lumineux desquels est interposé un miroir semi-réfléchissant 9 qui laisse passer les rayons lumineux vers un miroir sphérique concave 12 permettant d'éloigner l'image à distance. La voie de vision de loin 13 comporte, à partir d'une ouverture 14 dans le corps 1, un miroir semi-réfléchissant 15 qui renvoie le faisceau lumineux issu de l'image en vision de loin 16 sur un miroir plan 17, après réflexion sur un miroir concave 18.

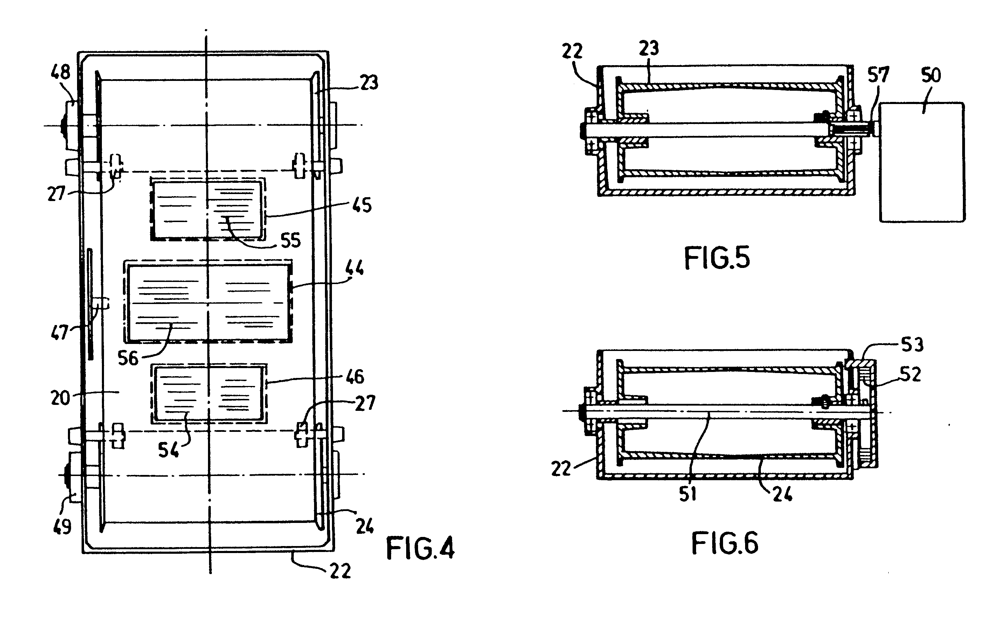

Les images-tests 51 pour la vision de près, 19 pour la vision à distance intermédiaire (de l'ordre de 0,7 mètre), et 16 pour la vision de loin, sont portées par le film ou le ruban 20 d'une cassette 21 dans le corps 22 de laquelle sont montées deux bobines d'enroulage 23 et 24 portant le film 20 et des moyens d'éclairage de ce film 20 tels que des lampes 25 alimentées en courant électrique par des prises et/ou bornes embrochables 26 qui coopèrent avec des bornes et/ou prises correspondantes du corps 1 de l'appareil. La cassette 21 représentée plus en détail aux figures 4 à 6 comporte également des rouleaux tendeurs de film 27 et des moyens d'accrochage et/ou de fixation non représentés permettant de la fixer de façon amovible sur un support du corps 1 de l'appareil.

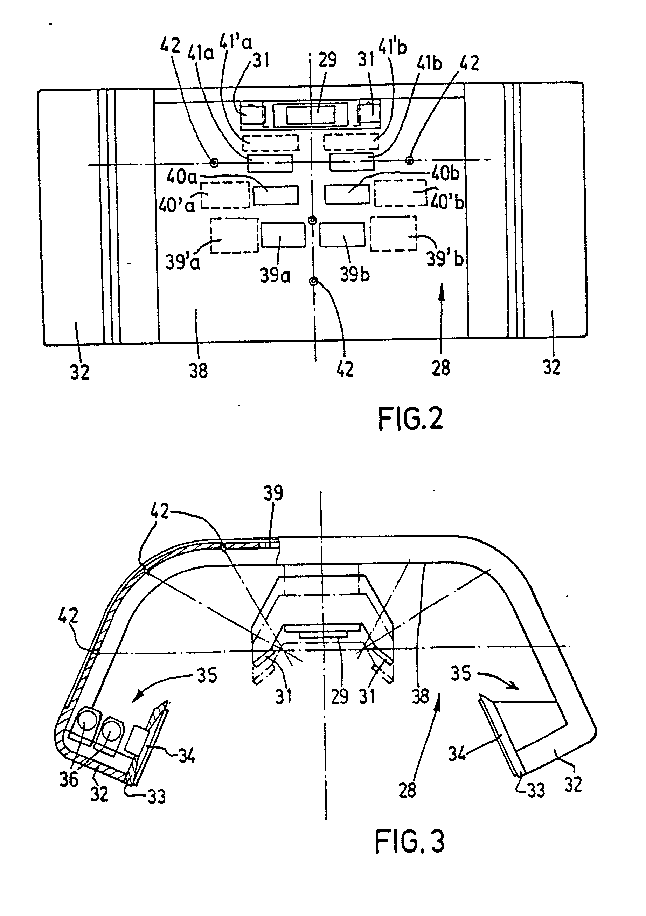

Sur la gauche, selon la figure 1, le corps 1 de l'appareil de test de la vue selon l'invention, présente une chambre de vision 28 qui, si l'on se reporte aux figures 2 et 3, présente en coupe transversale la forme générale d'un U très ouvert. Au centre de la chambre de vision 28 est disposé un appui-tête réglable, constitué d'une partie centrale 29 formant appui frontal pour le front du patient, en principe non-réglable par le patient mais réglé avant essai par une vis freinée 30 (cf. la fig. 1). L'appui-tête comporte deux appuis latéraux 31 pouvant s'appliquer sur les côtés du front ou les tempes du patient, et en principe réglables par celui-ci.

La chambre de vision 28 qui se referme par des rebords latéraux 32 sur les côtés rapportés 33 desquels on peut venir disposer des haut-parleurs 34 permettant de donner des séries enregistrées d'instructions au patient, forme des sortes de placards latéraux d'éclairage 35 dans lesquels on dispose un éclairage d'ambiance réglable constitué, par exemple, par des tubes fluorescents 36.

Sur sa face avant 38, la chambre de vision 28 présente trois séries de couples d'ouvertures 39 à 41, respectivement (40a, 40b) pour l'entrée de la voie de vision 6 à distance intermédiaire, (41a, 41b) pour l'entrée de la voie de vision 13 en vision de loin, et (39a, 39b) pour l'entrée de la voie de vision 2 en vision de près. Chacune des ouvertures de ces couples 39 à 41 peut être obturée par un rideau ou un volet (39'a, 39'b; 40'a, 40'b; 41'a, 41'b) tel que représenté en pointillés sur la figure 2 et placé sous la commande d'un opérateur ou programmé par des instructions d'obturation. La face 38 de la chambre de vision 28 comporte, réparties géométriquement en des points choisis autour des couples d'ouvertures 39 à 41, des émetteurs ponctuels ou quasi-ponctuels de lumière 42, tels que des diodes émettrices de lumière LED permettant de détecter d'éventuels défauts de champ de chacun des yeux du patient.

Si l'on se reporte à la figure 4, on voit que la plaque frontale de la cassette amovible 21 portant la batterie d'images-tests présente des ouvertures 44, 45 et 46 correspondant aux ouvertures 3, 7 et 14 du corps 1 de l'appareil et permettant la vision des images-tests via les trois voies de vision 2, 6 et 13 (les ouvertures sont représentées en pointillés).

Les ouvertures 44 à 46 sont plus grandes que les ouvertures 3, 7 et 14 du corps 1 qu'elles recouvrent entièrement en service. Par ces ouvertures 44 à 46, on peut contrôler l'état des images-tests lorsque la cassette est enlevée de l'appareil. Une ouverture 47 ménagée dans le corps frontal de la cassette permet à une cellule non représentée de l'appareil, de contrôler la position du film 20 des images-tests et, notamment, sa mise en service.

La série des questions posées au patient peut figurer sur une bande de magnétophone se déroulant en synchronisme avec le film 20 qui est mis en tension par les rouleaux tendeurs 27 et peut être déroulé par des moyens moteurs externes à la cassette et couplés aux bobines de celle-ci par des têtes de couplage latérales 48 et 49.

Selon un autre mode de réalisation représenté sur les figures 5 et 6, l'une des bobines, par exemple la bobine 23 de la cassette 21, est reliée mécaniquement par un entraînement irréversible à un moteur électrique 50, qui est, soit inclus dans la cassette 21 et alimenté par un couplage électrique non représenté, soit solidaire de l'appareil et couplé à la bobine au moment de la mise en place de la cassette 21. L'autre bobine 24 montée sur des paliers tels que des roulements à billes, est couplée par son axe 51 à un fort ressort spiral 52 accroché à une enveloppe 53 fixée au corps 22 de la cassette. La bobine 24 est ainsi rappelée constamment en position bobinée par le ressort spiral 52 et tend constamment le film 20. Lorsque le moteur électrique 50 tourne, il entraîne en rotation, par exemple dans le sens du bobinage, la bobine 23 qui tire sur le film 20 qui fait tourner la bobine 24 à l'encontre du ressort spiral 52. Dès que le moteur électrique 50 n'est plus sous tension, l'arbre de sortie 57 de sa transmission s'immobilise par suite du blocage de la transmission irréversible et la bobine 23 s'immobilise. Le ressort spiral 52 maintient le film 20 sous tension et les images-tests 54, 55 et 56 respectivement pour la vision de loin, à distance intermédiaire et la vision de près s'immobilisent, parfaitement planes et nettes. En mettant le moteur électrique 50 sous tension inverse, on inverse son sens de rotation, ce qui permet au ressort spiral 52 de rappeler la bobine 24 dans le sens du bobinage sous le contrôle de vitesse de la transmission irréversible. Au démontage de la cassette et au découplage du moteur 50, le film 20 est rappelé en position bobinée par le ressort spiral 52.

Le fonctionnement de l'appareil va maintenant être explicité. Un patient dispose sa tête en bonne position dans la chambre de vision 28 à l'aide des appuis latéraux 31 réglables et déclenche, à l'aide d'un bouton de départ non représenté, le cycle de la cassette 21.

Des images-tests successives viennent alors devant ses yeux en vision de près, en vision de loin et en vision intermédiaire, tandis que les haut-parleurs 34 lui donnent des instructions et l'interrogent sur sa vision de ces images-tests. A l'aide de boutons de commande *ad hoc*, le patient peut interrompre l'examen en des points déterminés, ou même revenir sur des phases passées de l'examen. Ses réponses aux questions sont enregistrées sur une bande de magnétophone qui, le test terminé, peut ultérieurement être écoutée à loisir par un opérateur qui pourra contrôler si les réponses aux questions posées correspondent bien aux tests présentés et, en cas d'anomalie, conseillera au patient de consulter un ophtalmologiste pour un examen plus approfondi.

Les réactions du patient à l'éclairage d'ambiance par les placards lumineux 35 et aux éclairs ponctuels des diodes 42 peuvent également être enregistrées sur la cassette 21.

En variante, une imprimante peut être reliée à l'appareil, imprimante qui permet de restituer sous forme de fiche ou carte perforée les réponses du patient.

Grâce à l'adaptation des miroirs sphériques 12 et 18, il est possible de réaliser sur le mêmes images l'examen en vision à distance intermédiaire et en vision de loin. Dans le cas où la personne qui désire examiner sa vue, par exemple un enfant, ne peut utiliser elle-même l'appareil, un opérateur contrôle le déroulement du film 20 de la cassette 21, pose lui-même les questions et enregistre ou note les réponses du patient. La réticence des patients à procéder régulièrement à un examen approfondi de leur vue est grandement réduite grâce à l'appareil selon l'invention, car non seulement le coût du dépistage est réduit, mais surtout le patient a l'impression de procéder lui-même, et à son rythme, à ce dépistage.

Selon un mode de réalisation plus élaboré de l'appareil, la cassette 21, avec ses deux bobines d'enroulage 23, 24, qui est représentée en position verticale par rapport au patient sur la figure 1, peut être disposée horizontalement selon une position d'accrochage auxiliaire qui fait défiler les images à visionner par le patient en face de l'ouverture 3. Dans la disposition qui est décrite sur les figures 1 et 2, le patient voit les images disposées verticalement ou bien défiler dans un plan vertical, tandis que dans la disposition auxiliaire horizontale de la cassette 21, l'opérateur peut faire défiler les images-tests horizontalement pour tester l'aptitude du patient à visionner des images en défilement horizontal.

Pour permettre des orientations diverses de la cassette 21, il est avantageux de l'équiper de son propre moteur électrique d'entraînement 50 fixé rigidement à la cassette et relié par un câble souple ou une connection à balais et contacts glissants à une alimentation électrique réglable pour permettre de régler selon les désirs du patient ou de l'opérateur de l'appareil, la vitesse de défilement et/ou l'arrêt du film 20. Il est ainsi possible de prévoir une cassette 21 fixée sur le corps 1 de l'appareil mais orientable en toutes directions dans un plan de déplacement et de défilement du film 20.

## Revendications

1. Appareil d'autotest pour l'examen de la vue des deux yeux d'un patient et l'auto-dépistage de ses défaillances visuelles sans intervention d'un opérateur, caractérisé en ce qu'il comporte des miroirs et/ou lentilles interposés sur au moins trois voies de vision (2, 6, 13) distinctes, chaque voie étant définie par ses propres dispositifs optiques, ces voies de vision (2, 6, 13) comprenant une voie pour l'examen de la vision de loin (13), au moins une voie pour l'examen de la vision intermédiaire (6) et une voie pour l'examen de la vision de près (2), et en ce qu'il comporte une chambre de vision (28) munie d'un appui tête réglable (29, 31) pour le positionnement du visage d'un patient, lequel appui permet au patient par balayage vertical du regard, l'observation successive des trois images-tests (5, 16, 19), appartenant chacune à une des trois voies de vision.

2. Appareil selon la revendication 1, caractérisé en ce que à l'entrée dans la chambre de vision les voies de vision sont placées de façon telle que la voie pour l'examen de la vision de loin soit la plus haute, la voie pour l'examen de la vision de près soit la plus basse et la voie pour l'examen de la vision intermédiaire soit en position intermédiaire.

3. Appareil selon la revendication 1 ou 2, caractérisé en ce que les images-tests (5, 16, 19) sont portées par au moins un ruban ou un film (20) monté dans un bloc à deux bobines d'enroulage (23, 24) logé dans une cassette (21) fixée de façon amovible sur un support du corps (1) de l'appareil, au moins une des bobines étant susceptible d'être entraînée en rotation pour faire défiler une batterie d'images-tests (54, 55, 56) sur une partie du ruban ou du film (20), qui est tendue entre les deux bobines (23, 24) et qui se place en face d'ouvertures (3, 7, 14) d'amenée des images-tests ménagées sur le corps de l'appareil.

4. Appareil selon l'une quelconque des revendications 1 à 3, caractérisé en ce que la voie pour l'examen de la vision de loin (13) et la ou les voies pour l'examen de la vision à distance intermédiaire (6) sont équipées de miroirs sphériques concaves (18, 12) permettant la vision d'une même image-test indifféremment sur chacune de ces voies.

5. Appareil selon l'une quelconque des revendications 1 à 4, caractérisé en ce que pour chacune des voies de vision (2, 6, 13) l'appareil comporte, du côté du patient, deux ouvertures, chaque ouverture (39a, 39b; 40a, 40b; 41a, 41b) de chaque voie étant susceptible d'être occultée par un moyen d'obturation tel qu'un volet mobile (39'a, 39'b; 40'a, 40'b; 41'a, 41'b).

6. Appareil selon l'une quelconque des revendications 1 à 5, caractérisé en ce qu'il comporte des moyens pour immobiliser la rotation des bobines (23, 24) de la cassette (21) susceptible d'être commandés par le patient pendant le défilement de la batterie d'images-tests (54—56) et des moyens pour enregistrer les réponses du patient.

7. Appareil selon l'une quelconque des revendications 1 à 6, caractérisé en ce qu'il comporte entre les ouvertures d'amenée des images-tests des sources ponctuelles ou quasi-ponctuelles de lumière, telles que des diodes LED (42), permettant de détecter les défauts de champ visuel du patient et les défaillances ponctuelles éventuelles de sa rétine.

8. Appareil selon l'une quelconque des revendications 1 à 7, caractérisé en ce que ladite chambre de vision (28) recevant la tête du patient est équipée d'un appui réglable (29, 31) pour le front du patient lui permettant de maintenir en cours d'examen le centre de rotation de chacun de ses yeux confondu avec le point de convergence (0) des voies de vision (2, 6, 13) correspondant à chaque oeil.

9. Appareil selon l'une des revendications 1 à 8, caractérisé en ce que le ruban ou le film (20) de la cassette (21) est susceptible d'être entraîné en défilement à une vitesse réglable afin de permettre de tester l'aptitude du patient à visionner des images défilantes.

10. Appareil selon l'une des revendications 1 à 9, caractérisé en ce que la cassette amovible (21) portant le ruban ou le film (20) est fixée sur le corps (1) de l'appareil dans une position faisant défiler ce ruban ou le film (20) dans une direction sensiblement verticale.

11. Appareil selon l'une des revendications 1 à 9, caractérisé en ce que la cassette amovible portant le ruban ou le film (20) est fixée sur le corps (1) de l'appareil dans une position faisant défiler ce ruban (20) dans une direction sensiblement horizontale.

12. Appareil selon l'une des revendications 1 à 11, caractérisé en ce que la cassette amovible (21) portant le ruban ou le film (20) est susceptible d'être fixée sur le corps (1) de l'appareil alternativement dans deux positions perpendiculaires permettant de faire défiler le ruban (20) pour l'une dans une direction sensiblement verticale, et pour l'autre, dans une direction sensiblement horizontale.

13. Appareil selon l'une des revendications 1 à 12, caractérisé en ce que la cassette amovible (21) portant le ruban (20) et fixée sur le corps (1) de l'appareil est susceptible d'être orientée en une pluralité de positions dans un plan de déplacement du ruban ou du film (20) de manière à permettre de faire défiler le ruban ou le film (20) selon une pluralité de directions correspondantes comportant les directions verticales et horizontales ainsi qu'une pluralité de directions intermédiaires et en ce que, afin de permettre le défilement du ruban (20) dans toutes les directions, la cassette (21) porte son propre moteur (50) d'entraînement du ruban ou du film (20) qui est relié à un moyen de contrôle réglable à distance.

## Patentansprüche

1. Selbstprüfgerät zum Prüfen des Sehvermögens der beiden Augen eines Patienten und zur selbständigen Ermittlung seiner Sehfehler ohne Zutun einer Behandlungsperson, dadurch gekennzeichnet, dass es Spiegel und/oder Linsen aufweist, die auf wenigstens drei verschiedenen Sichtlinien (2, 6, 13) angeordnet sind, wobei jede Sichtlinie durch ihre eigenen optischen Vorrichtungen bestimmt ist und diese Sichtlinien (2, 6, 13) eine Sichtlinie (13) für die Prüfung der Weitsicht, wenigstens eine Sichtlinie (6) für die Prüfung der Mittelsicht und eine Sichtlinie (2) für die Prüfung der Nahsicht umfassen, und dass das Gerät eine Sichtkammer (28) aufweist, die mit einer einstellbaren Kopfstütze (29, 31) zum Positionieren des Gesichts des Patienten versehen ist, derart, dass diese Kopfstütze es dem Patienten gestattet, durch senkrechte Blickabstastung die drei den drei Sichtlinien jeweils zugeordneten Prüfbilder (5, 16, 19) nacheinander zu beobachten.

2. Gerät nach Anspruch 1, dadurch gekennzeichnet, dass die Sichtlinien am Eingang der Sichtkammer derart angeordnet sind, dass die Linie für die Weitsichtprüfung am höchsten liegt, während die Linie für die Prüfung der Nahsicht am tiefsten liegt und die Linie für die Prüfung der Mittelsicht sich in einer Zwischenlage befindet.

3. Gerät nach Anspruch 1 oder 2, dadurch gekennzeichnet, dass die Prüfbilder (5, 16, 19) von wenigstens einem Band (oder Film) (20) getragen werden, das in einem in einer Kassette (21) eingebauten Block mit zwei Wickelspulen (23, 24) angeordnet ist, wobei die Kassette lösbar an einem Träger des Gerätegehäuses (1) angebracht ist und wenigstens eine der Spulen gedreht werden kann, um eine Gruppe von Prüfbildern (54, 55, 56) auf dem Band (bzw. Film) (20) durchlaufen zu lassen, das zwischen den beiden Spulen gespannt ist und sich vor im Gerätegehäuse angeordneten Prüfbildeingangsöffnungen (3, 7, 14) erstreckt.

4. Gerät nach einem der Ansprüche 1 bis 3, dadurch gekennzeichnet, dass die Weitsichtprüflinie (13) und die Mittelsichtprüflinie(n) (6) mit konkaven sphärischen Spiegeln versehen sind, die es gestatten, ein und dasselbe Prüfbild auf jeder dieser Linien zu beobachten.

5. Gerät nach einem der Ansprüche 1 bis 4, dadurch gekennzeichnet, dass es für jede Sichtprüflinie (2, 6, 13) auf der dem Patienten zugewandten Seite zwei Öffnungen besitzt, wobei jede Öffnung (39a, 39b; 40a, 40b; 41a, 41b) einer jeden Sichtlinie durch ein Verschlussmittel, wie eine bewegliche Klappe (39'a, 39'b; 40'a, 40'b; 41'a, 41'b) verschliessbar ist.

6. Gerät nach einem der Ansprüche 1 bis 5, dadurch gekennzeichnet, dass es Mittel aufweist, durch welche die Spulen (23, 24) der Kassette (21) drehfest gehalten werden können und die vom Patienten während des Durchgangs der Prüfbildergruppe (54—56) betätigbar sind, sowie Mittel zum Aufnehmen der Antworten des Patienten.

7. Gerät nach einem der Ansprüche 1 bis 6, dadurch gekennzeichnet, dass es zwischen den Eingangsöffnungen für die Prüfbilder punktförmige bzw. annähernd punktförmige Lichtquellen, wie LED-Dioden (42) besitzt, wodurch die Sehbereichsfehler des Patienten und ggf. lokale Fehler seiner Netzhaut feststellbar sind.

8. Gerät nach einem der Ansprüche 1 bis 7, dadurch gekennzeichnet, dass die den Kopf des Patienten aufnehmende Sichtkammer (28) eine einstellbare Stütze (29, 31) für die Stirn des Patienten besitzt, derart, dass letzterer während der Sichtprüfung das Drehzentrum eines jeden seiner Augen im Schnittpunkt (10) der Sichtlinien (2, 6, 13) des jeweiligen Auges halten kann.

9. Gerät nach einem der Ansprüche 1 bis 8, dadurch gekennzeichnet, dass das Band (bzw. Film) (20) der Kassette (21) mit einer einstellbaren Durchgangsgeschwindigkeit antreibbar ist, sodass festgestellt werden kann, welche Fähigkeit der Patient besitzt, die durchgehenden Bilder zu beobachten.

10. Gerät nach einem der Ansprüche 1 bis 9, dadurch gekennzeichnet, dass die das Band (bzw. Film) (20) tragende abnehmbare Kassette (21) am Gerätegehäuse (1) in einer solchen Lage angeordnet ist, dass dieses Band (bzw. Film) (20) in wesentlich vertikaler Richtung durchläuft.

11. Gerät nach einem der Ansprüche 1 bis 9, dadurch gekennzeichnet, dass die das Band (bzw. Film) (20) tragende abnehmbare Kassette in einer solchen Lage am Gerätegehäuse (1) angeordnet ist, dass dieses Band (20) in wesentlich horizontaler Richtung durchläuft.

12. Gerät nach einem der Ansprüche 1 bis 11, dadurch gekennzeichnet, dass die das Band (bzw. Film) (20) tragende abnehmbare Kassette (21) am Gerätegehäuse (1) wechselweise in zwei zueinander senkrechten Lagen anbringbar ist, um das Band (20) in der einen Lage in wesentlich vertikaler Richtung und in der anderen Lage in wesentlich horizontaler durchlaufen zu lassen.

13. Gerät nach einem der Ansprüche 1 bis 12, dadurch gekennzeichnet, dass die das Band (20) tragende, abnehmbar am Gerätegehäuse (1) angeordnete Kassette (21) in eine Vielzahl von Lagen bringbar ist, die in einer Bewegungsebene des Bandes (bzw. Films) (20) definiert sind, sodass das Band (bzw. Film) (20) in einer Vielzahl entsprechender Richtungen, einschliesslich der vertikalen und horizontalen Richtung und einer Anzahl von Zwischenrichtungen, durchlaufen kann, und ferner dadurch gekennzeichnet, dass die Kassette (21) ihren eigenen Motor (50) zum Antrieb des Bandes (bzw. Films) (20) trägt, um ein Durchlaufen des letzteren in allen Richtungen zu gestatten, wobei der Motor mit Fernsteuermitteln verbunden ist.

## Claims

1. Apparatus for self-testing the vision of the two eyes of a patient and for self-detecting his vision defects without the aid of an operator, characterized in that it comprises mirrors and/or lenses interposed on at least three distinct vision paths (2, 6, 13) each of which is defined by its particular optical device, said vision paths (2, 6, 13) comprising one path (13) for testing the far vision, at least one path (6) for testing the intermediate vision and one path (2) for testing the near vision, and in that it comprises a vision chamber (28) provided with an adjustable head rest (29, 31) for positioning the face of a patient, said head rest enabling the patient to observe successively by vertical visual scanning the three test images (5, 16, 19) associated each to one of said vision paths.

2. Apparatus according to claim 1, characterized in that the vision paths are positioned at the inlet of the vision chamber in such a manner that the vision path for testing the far vision is located at the uppermost position, the vision path for testing the near vision is located at the lowermost position and the vision line for testing the intermediate vision is located at an intermediate position.

3. Apparatus according to claim 1 or 2, characterized in that the test images (5, 16, 19) are carried on at least one ribbon or film (20) mounted in a block with two winding coils (23, 24) arranged within a cassette (21) which is removably fixed onto a support of the body (11) of said apparatus, at least one of said coils being adapted to be rotatively driven so as to cause a series of test images (54, 55, 56) on a portion of the ribbon or film (20) to pass, which portion is extending between the two coils (23, 24) and moves in front of test image inlet openings (3, 7, 14) provided on said body of the apparatus.

4. Apparatus according to any one of claims 1 to 3, characterized in that the far vision testing path (13) and the intermediate vision testing path or paths (6) are provided with concave spherical mirrors (18, 12) allowing one given test image to be viewed indifferently on any one of said paths.

5. Apparatus according to any one of claims 1 to 4, characterized in that it comprises for each one of said vision paths (2, 6, 13) on the side of the patient two openings, each opening (39a, 39b; 40a, 40b; 41a, 41b) of each path being adapted to be closed by a closure means such as a movable flap (39'a, 39'b; 40'a, 40'b; 41'a, 41'b).

6. Apparatus according to any one of claims 1 to 5, characterized in that it comprises means for blocking said coils (23, 24) of said cassette (21) against rotation, said means being adapted to be controlled by the patient during the passage of the series of test images (54—56), and means for recording the responses of the patient.

7. Apparatus according to any one of claims 1 to 6, characterized in that it comprises, between the inlet openings for the test images, point-shaped or substantially point-shaped light sources, such as LED diodes (42) allowing the detection of defects of the visual field of the patient and detection of possible localized defects of his retina.

8. Apparatus according to any one of claims 1 to 7, characterized in that said vision chamber (28) receiving the head of the patient is provided with an adjustable rest (29, 31) for the front of the patient so as to enable him or her to maintain during the testing procedure the center of rotation of each one of his or her eyes in coincidence with the point of intersection (0) of the vision paths (2, 6, 13) corresponding to each eye.

9. Apparatus according to any one of claims 1 to 8, characterized in that the ribbon of film (20) of said cassette (21) is adapted to be driven so as to travel at an adjustable speed, and thus to allow the patient's aptitude of viewing the passing images to be tested.

10. Apparatus according to any one of claims 1 to 9, characterized in that said removable cassette (21) carrying the ribbon of film (20) is fixed onto the body (1) of the apparatus in a position so selected that the said ribbon of film (20) is displaced in a substantially vertical position.

11. Apparatus according to any one of claims 1 to 9, characterized in that said removable cassette (21) carrying the ribbon or film (20) is fixed onto the body (1) of the apparatus in a position so selected that said ribbon (20) is displaced in a substantially horizontal direction.

12. Apparatus according to any one of claims 1 to 11, characterized in that the removable cassette (21) carrying the ribbon or film (20) is adapted to be mounted onto the body (1) of said apparatus alternatively in two respective perpendicular positions, so as to cause the ribbon (20) to be displaced, in the case of one of said positions, in a substantially vertical direction and, in the case of the other position, in a substantially horizontal direction.

13. Apparatus according to any one of claims 1 to 12, characterized in that the removable cassette (21) carrying the ribbon (20) and affixed to the body (1) of said apparatus is adapted to be oriented in accordance with a plurality of positions in a plane of displacement of said ribbon or film (20), so as to allow the ribbon or film (20) to travel in a plurality of directions comprising the vertical direction, the horizontal direction and a plurality of intermediate directions, and in that with a view to allowing the ribbon (20) to be displaced in all directions said cassette (21) carries its own motor (50) for driving the ribbon or film (20), said motor being connected to remote control means.

FIG.1

0 102 887

FIG.2

FIG.3

2

FIG.4

FIG.5

FIG.6

0 102 887